# EUROPÄISCHE PATENTSCHRIFT

(11) **EP 2 805 739 B1**
(45) Veröffentlichungstag und Bekanntmachung des Hinweises auf die Patenterteilung: **11.07.2018**
(21) Anmeldenummer: 14168100.7
(22) Anmeldetag: 13.05.2014
(51) Int. Cl.: A61B 5/00, G01F 3/20

(54) **Vorrichtung, Anordnung und Verfahren zur Messung eines Volumenstroms eines in einer Leitung strömenden Fluids**
Device, assembly and method for measuring a volume flow of a fluid flowing within a pipe
Dispositif, agencement et procédé de mesure d'un débit volumique d'un fluide s'écoulant dans une conduite

(30) Priorität: 24.05.2013 DE 102013209687
(43) Veröffentlichungstag der Anmeldung: 26.11.2014
(73) Patentinhaber: Spiegelberg GmbH & Co. KG, 21079 Hamburg (DE)
(72) Erfinder: Dr. Stieglitz, Lennart, 8052 Zürich (CH)
(74) Vertreter: Maikowski & Ninnemann Patentanwälte Partnerschaft mbB

(56) Entgegenhaltungen:
- GB-A- 920 694
- US-A- 488 504
- US-A- 4 660 568

## Beschreibung

Die Erfindung betrifft eine Vorrichtung zur Messung eines Volumenstroms eines in einer Leitung strömenden Fluids gemäß Anspruchs 1, ein eine solche Vorrichtung aufweisende Anordnung zur Messung eines Volumenstroms eines in einer Leitung strömenden Fluids gemäß Anspruchs 13 und ein Verfahren zur Messung eines Volumenstroms eines in einer Leitung strömenden Fluids gemäß Anspruchs 15. Dabei handelt es sich bei der Leitung insbesondere um eine medizinische Drainageleitung. Ferner handelt es sich bei dem Fluid insbesondere um eine Körperflüssigkeit und ganz besonders um Hirnwasser (Liquor).

Für die Behandlung von Patienten, bei denen ein Ungleichgewicht zwischen der Bildung von Hirnwasser und der Rückresorption von Hirnwasser besteht, den sogenannten Hydrozephaluspatienten, werden Drainageeinrichtungen verwandt. Vollständig implantierbare Drainageeinrichtungen bestehen üblicherweise aus einem Ventrikelkatheter, der mit seiner Spitze in der Hirnkammer des Patienten platziert wird und unter der Haut zu einem Ventil geführt wird, einem Ventil, das den Rückstrom von Hirnwasser verhindert und eine Überdruckfunktion hat, und einem peripheren Katheter, der den Auslass des Ventils mit einem Hohlraum des Körpers, zum Beispiel der Bauchhöhle verbindet. Für derartige Einsatzzwecke geeignete Ventile sind entweder mit einem festen Ansprechdruck, der für den Patienten passend individuell auswählbar ist, ausgestattet, oder sie sind einstellbar. Derartige Drainageeinrichtungen werden auch als Shuntsysteme bezeichnet.

Wenn allerdings bei einem Hydrozephaluspatienten mit einem Shuntsystem Probleme auftreten, müssen immer sehr aufwendige, teure und oft risikoreiche Untersuchungen durchgeführt werden. Ein großer Anteil der Shuntsysteme wird im Laufe des Lebens zwar unbrauchbar, aber ein Nachweis des Versagens im implantierten Zustand ist bisher noch nicht zuverlässig möglich.

Nach dem heutigen Stand der Technik werden, wenn ein Patient mit einem Shuntsystem Probleme entwickelt, folgende Untersuchungsverfahren angewandt:

### a) Pumpen des Shuntsystems

Eine in den Shunt zwischen Ventrikelkatheter und Ventil eingebaute Pumpkammer wird durch die Haut hindurch manuell komprimiert. Dabei wird der Katheter entweder vor oder hinter der Pumpkammer zusammengedrückt, so dass zu spüren ist, ob das Ventil oder der Ventrikelkatheter verschlossen ist. Dieses Verfahren ist zunächst davon abhängig, dass überhaupt eine Pumpkammer in das Shuntsystem integriert ist. Es ist ferner sehr von der Erfahrung des untersuchenden Arztes abhängig und insgesamt ungenau.

### b) Operative Revision des Systems

Dabei wird in einer chirurgischen Operation das gesamte Shuntsystem oder Teile davon entfernt und durch eine neues Shuntsystem oder neue Teile ersetzt. Dies ist ein aufwändiges, kostenträchtiges und für den Patienten sehr belastendes Verfahren.

### c) Computertomographie (CT)

Dabei wird ein Schichtbild des Schädels angefertigt und versucht, aus der Morphologie der Hirnkammern Rückschlüsse auf die Funktionsfähigkeit des Shunts zu ziehen. Auch dies ist ein sehr belastendes (Strahlenbelastung), aufwändiges und nicht sehr treffsicheres Verfahren.

### d) Telemetrische Druckmessung

Wenn ein telemetrisches Druckmesssystem Einsatz findet, wird der momentan herrschende Druck ausgelesen. Es kann der Druck-Mittelwert oder die kurzzeitige Druck-Wellenform betrachtet werden. Das Verfahren und die Einrichtung sind sehr aufwändig und teuer. Außerdem kann durch eine Druckmessung, die grundsätzlich durch viele physiologische Einflüsse überlagert ist, nicht direkt auf die Funktionsfähigkeit des Ventils und der Katheter eines Shuntsystems geschlossen werden.

Die bisher üblichen Verfahren sind also mit erheblichen Nachteilen für die betreffenden Patienten behaftet, die nachfolgend nochmals zusammengefasst werden:

### Aufwand und Kosten:

Alle derzeit möglichen Untersuchungsmethoden sind mit hohem Aufwand und hohen Kosten verbunden.

### Operationsrisiko:

Wenn eine eindeutige Diagnose von außen nicht möglich ist, bleibt meistens nur der operative Eingriff mit allen Risiken und Belastungen für den Patienten als Alternative.

### Strahlenbelastung:

Die Strahlenbelastung durch CT-Untersuchungen ist erheblich.

### Diagnoseungenauigkeiten und Fehldiagnosen:

Alle derzeit möglichen Diagnosemethoden bergen Risiken der Ungenauigkeit. Selbst die zurzeit bestehenden "einfachen" telemetrischen Druckmesssysteme können nur unvollständige Daten in Form von Mittelwerten oder kurzzeitigen Kurvenformen übertragen. Verfahren und Einrichtungen zur nichtinvasiven Bestimmung des Durchflusses solcher Shuntsysteme sind bisher nicht bekannt.

Aus der US 2005/0155424 A1 und der US 488,504 A sind Gasmessvorrichtungen gemäß dem Oberbegriff des Anspruchs 1 bekannt. US4660568 beschreibt einen Drucksensor. GB920694 beschreibt einen Durchflussmesser, der zum Messen geringer Volumina geeignet ist. Der vorliegenden Erfindung liegt die Aufgabe zugrunde, eine Vorrichtung zur Messung eines Volumenstroms eines in einer Leitung strömenden Fluids bereitzustellen, die sich zur Messung des Volumenstroms von Hirnwasser in einem Ventrikelkatheter eignet sowie einfacher und weniger aufwändig zu bedienen ist als aus dem Stand der Technik bekannte Vorrichtungen. Diese Aufgabe wird mit einer Vorrichtung mit den Merkmalen des Anspruchs 1 gelöst. Eine derartige Vorrichtung zur Messung eines Volumenstroms eines in einer Leitung strömenden Fluids weist ein Gehäuse auf, das durch eine Gehäusewand gebildet ist. In der Gehäusewand ist ein Einlass ausgebildet, durch den hindurch ein Fluid aus einem ersten Abschnitt einer Leitung in das Gehäuse eintreten kann. Bei dieser Leitung kann es sich beispielsweise um einen Ventrikelkatheter handeln. Ferner ist in der Gehäusewand ein Auslass gebildet, durch den hindurch ein Fluid aus dem Gehäuse austreten kann. Dabei kann das Fluid durch den Auslass in einen zweiten Abschnitt der Leitung treten oder beispielsweise direkt in ein Sammelgefäß überführt werden.

In dem Gehäuse ist ferner eine gegenüber der Gehäusewand zumindest abschnittsweise bewegliche Wand angeordnet, die fluiddicht ausgestaltet ist. Diese Wand unterteilt das Gehäuse in eine erste Kammer und in eine zweite Kammer und verhindert auf diese Weise einen direkten Übertritt eines in dem Gehäuse befindlichen Fluids von der ersten Kammer in die zweite Kammer und umgekehrt.

In dem Gehäuse sind zudem ein erstes und ein zweites Ventil angeordnet, die miteinander in Wirkverbindung stehen, so dass eine Überführung des ersten Ventils oder des zweiten Ventils von seiner geöffneten Stellung in seine geschlossene Stellung gleichzeitig bewirkt, dass das jeweils andere Ventil von seiner geschlossenen Stellung in seine geöffnete Stellung überführt wird. Ferner wird durch die Wirkverbindung zwischen dem ersten Ventil und dem zweiten Ventil sichergestellt, dass auch eine Überführung des ersten Ventils und/oder des zweiten Ventils von seiner geschlossenen Stellung in seine geöffnete Stellung eine gleichzeitige Überführung des jeweils anderen Ventils von seiner geöffneten Stellung in seine geschlossene Stellung bewirkt. Mit anderen Worten ausgedrückt, sind das erste Ventil und das zweite Ventil derart miteinander gekoppelt, dass das eine Ventil immer dann vollständig geöffnet ist, wenn das andere Ventil vollständig geschlossen ist. Bei der Überführung des einen oder des anderen Ventils von seiner geöffneten Stellung in seine geschlossene Stellung können kurzzeitig beide Ventile geöffnet sein. In diesem intermediären Zustand sind die Ventile jedoch nicht vollständig geöffnet, sondern lediglich teilweise geöffnet. Mit Ausnahme dieser sehr kurzen Schaltzeiten sind das erste Ventil und das zweite Ventil daher stets wechselseitig geöffnet und geschlossen. Die Ventile sind dabei derart ausgebildet, dass ein Schließen und/oder Öffnen des ersten Ventils und/oder des zweiten Ventils ein detektierbares Signal erzeugt, das heißt, mit einem geeigneten Detektor lässt sich erkennen, ob gerade ein Öffnungs- oder Schließvorgang des ersten Ventils und/oder des zweiten Ventils stattfindet bzw. begonnen oder beendet wird.

Ferner sind das erste Ventil und das zweite Ventil derart ausgelegt, dass eine Kraft, die zum Öffnen des zweiten Ventils benötigt wird, größer ist als eine Kraft, die zum Schließen des zweiten Ventils benötigt wird. Aufgrund der oben erläuterten Wirkverbindung zwischen dem ersten Ventil und dem zweiten Ventil ist die Kraft, die zum Schließen des zweiten Ventils benötigt wird, gleich der Kraft, die zum Öffnen des ersten Ventils benötigt wird. Man kann daher auch sagen, dass die Kraft, die zum Öffnen des zweiten Ventils benötigt wird, größer ist als eine Kraft, die zum Öffnen des ersten Ventils benötigt wird. Aufgrund dieses Ungleichgewichts der zum Öffnen und Schließen des zweiten Ventils benötigten Kraft ist es einfacher, das zweite Ventil zu schließen als es zu öffnen.

Durch die Wechselwirkung zwischen dem ersten Ventil und dem zweiten Ventil wird eine Ventilanordnung gebildet, die in zwei Stellungen vorliegen kann. In der ersten Stellung ist das erste Ventil geöffnet und das zweite Ventil geschlossen. In der zweiten Stellung ist das erste Ventil geschlossen und das zweite Ventil geöffnet. Aufgrund der unterschiedlichen Kräfte, die zum Öffnen und Schließen des zweiten Ventils erforderlich sind, befindet sich die Ventilanordnung bevorzugt in der ersten Stellung (also mit geöffnetem ersten Ventil und mit geschlossenem zweiten Ventil). Diese Stellung kann auch als stabile Stellung bezeichnet werden. Wenn eine ausreichend große Kraft auf die Ventilanordnung in Öffnungsrichtung des zweiten Ventils ausgeübt wird, springt die Ventilanordnung in die zweite Stellung (in der das erste Ventil geschlossen und das zweite Ventil geöffnet ist) um. In dieser Stellung verbleibt die Ventilanordnung jedoch nur solange, wie eine ausreichend große Kraft auf die Ventilanordnung ausgeübt wird. In dem Moment, in dem die Kraft unter einen definierten Wert sinkt, springt die Ventilanordnung automatisch wieder zurück in die erste Stellung. Daher kann die zweite Stellung auch als metastabile Stellung bezeichnet werden.

Schließlich steht die Wand, die das Gehäuse in die erste Kammer und in die zweite Kammer teilt, derart mit dem ersten und/oder dem zweiten Ventil in Wechselwirkung, dass die Kraft, die zum Öffnen des zweiten Ventils benötigt wird, durch eine Bewegung zumindest eines Abschnitts der Wand auf das erste und/oder das zweite Ventil übertragen wird. Mit anderen Worten ausgedrückt, sorgt eine Bewegung der Wand dafür, dass die Ventilanordnung von ihrer stabilen Stellung in ihre metastabile Stellung überführt werden kann. Die Bewegung der Wand erfolgt dabei vorzugsweise in Öffnungsrichtung des zweiten Ventils. Durch geeignete Kraftübertragungsmechanismen ist jedoch auch eine umgekehrte Bewegungsrichtung grundsätzlich denkbar.

Die beanspruchte Vorrichtung zur Messung eines Volumenstroms funktioniert nun derart, dass bei geöffnetem ersten Ventil ein Fluid durch den Einlauf in die erste Kammer einströmen kann. Dadurch füllt sich die erste Kammer, so dass die Wand gegenüber der Gehäusewand bewegt wird. Diese Bewegung der Wand erfolgt dabei vorzugsweise in Richtung der zweiten Kammer; es kommt also zu einer Ausdehnung der ersten Kammer. Durch die Wechselwirkung der Wand mit der Ventilanordnung bzw. mit dem ersten Ventil oder mit dem zweiten Ventil wird nun durch die Ausdehnung der ersten Kammer eine Kraft in Öffnungsrichtung des zweiten Ventils auf die Ventilanordnung übertragen, die zur Überführung der Ventilanordnung in die metastabile Stellung führt, wenn die zum Öffnen des zweiten Ventils benötigte Kraft überschritten wird. Mit anderen Worten ausgedrückt, kommt es zur Öffnung des zweiten Ventils, wenn die von der Wand ausgeübte Kraft größer als die Haltekraft des zweiten Ventils ist. Durch eine Öffnung des zweiten Ventils kann nun das Fluid aus der ersten Kammer in die zweite Kammer strömen. Dadurch bewegt sich die Wand wieder zurück in Richtung zur ersten Kammer.

Vorzugsweise ist die Wand derart ausgestaltet, dass eine Rückstellkraft auf sie wirkt, die entgegen der Richtung wirkt, in die die Kammer sich bewegt, wenn ein Fluid in die erste Kammer einströmt. Durch die entsprechende Rückwärtsbewegung der Wand wird die Kraft, die von der Wand auf die Ventilanordnung übertragen wird, geringer, bis sie kleiner ist als die zum Öffnen des zweiten Ventils erforderliche Kraft. Bei einem derart gelagerten Kräfteverhältnis springt die Ventilanordnung dann wieder von ihrer metastabilen Stellung in ihre stabile Stellung um; das zweite Ventil schließt und das erste Ventil öffnet. Anschließend kann ein neuer Zyklus der Befüllung der ersten Kammer, Umschalten der Ventilanordnung in ihre metastabile Stellung und anschließender Entleerung der ersten Kammer in die zweite Kammer erfolgen.

Wie aus den vorherigen Erläuterungen ersichtlich ist, stehen die erste Kammer und die zweite Kammer in Strömungsverbindung miteinander, wobei diese Strömungsverbindung unterbrochen werden kann. Die fluiddicht ausgestaltete Wand unterbindet dabei gerade eine Strömungsverbindung zwischen der ersten Kammer und der zweiten Kammer. Vielmehr erfolgt diese Strömungsverbindung vorzugsweise mittels des zweiten Ventils. In einer Variante ist das zweite Ventil daher zwischen der ersten Kammer und der zweiten Kammer angeordnet. Alternativ oder zusätzlich ist zudem in einer Variante das erste Ventil zwischen dem Einlass und der ersten Kammer angeordnet. Auf diese Weise lässt sich also durch das erste Ventil das Einströmen von Fluid in die erste Kammer steuern, während das zweite Ventil zur Steuerung der Fluidströmung von der ersten Kammer in die zweite Kammer dient.

Da die Kraftverhältnisse, die zum Öffnen/Schließen des ersten Ventils bzw. des zweiten Ventils vorbestimmbar sind, lässt sich auf diese Weise auch bestimmen, welches Fluidvolumen von der ersten Kammer in die zweite Kammer strömen kann, während das zweite Ventil geöffnet ist. Da das Schließen und/oder Öffnen des zweiten Ventils und/oder des zweiten Ventils ein detektierbares Signal erzeugt, kann dieses Signal also in direkte Korrelation mit einem Fluidvolumen gesetzt werden, das zwischen zwei Schaltvorgängen der Ventilanordnung von der ersten Kammer in die zweite Kammer strömen kann. Dieses Fluidvolumen ist identisch mit dem Fluidvolumen, das in der gleichen Zeiteinheit durch eine Leitung strömt, an die die beanspruchte Vorrichtung angeschlossen ist. Folglich kann aus der mittels des Signals detektierbaren Anzahl von Schaltvorgängen der Ventilanordnung pro Zeiteinheit direkt auf den Volumenstrom des in der entsprechenden Leitung strömenden Fluids geschlossen werden.

Grundsätzlich können die unterschiedlichsten Signale durch die Ventilanordnung bei ihrem Öffnen und/oder Schließen erzeugt werden. Denkbar sind beispielsweise eine Veränderung eines Magnetfelds, eine Veränderung eines elektrischen Feldes, ein optisches Signal oder ein elektrisches Signal. In einer bevorzugten Ausgestaltung der Erfindung handelt es sich bei dem Signal, das beim Öffnen und/oder Schließen des ersten Ventils und/oder des zweiten Ventils erzeugt wird, um ein akustisch detektierbares Signal. Beispielsweise kann durch die Ventilanordnung beim Öffnen und/oder Schließen eines der beiden Ventile ein Klicken erzeugt werden, das leicht detektiert werden kann. Zur Detektion eignet sich beispielsweise ein Mikrofon. Falls die Vorrichtung zur Messung eines Volumenstroms unter der Haut eines Patienten implantiert ist, ist auch der Einsatz eines Stethoskops zur Detektion eines entsprechenden akustischen Signals geeignet. Eine untersuchende Person würde dann das akustische Signal bzw. das Klicken hören können und die Anzahl der Umschaltvorgänge der Ventilanordnung pro Zeiteinheit ermitteln können. Wie erläutert, lässt sich aus der Anzahl der Umschaltvorgänge pro Zeiteinheit ohne Weiteres der Volumenstrom des die Vorrichtung durchströmenden Fluids berechnen.

In einer Variante handelt es sich bei dem ersten Ventil und/oder bei dem zweiten Ventil um Magnetventile. Dabei sind die Magnete der Magnetventile vorzugsweise derart gepolt, dass sie einander abstoßen, so dass die geöffnete Stellung die bevorzugte Stellung der Magnetventile ist. Grundsätzlich ist eine anziehende Polung auch denkbar. Magnetventile erzeugen insbesondere beim Schließen ein deutlich wahrnehmbares akustisches Signal, so dass in dieser Variante keine zusätzliche Signalerzeugungseinheit vorgesehen sein muss. Vielmehr wird das detektierbare Signal inhärent durch die Magnetventile selbst erzeugt.

In einer weiteren Variante sind die Ventile derart ausgeführt, dass sie unter Ausnutzung nicht magnetischer Kräfte geöffnet und geschlossen werden können. Hierzu eignet sich beispielsweise eine Federanordnung, die einzelne Bauteile des ersten Ventils und/oder des zweiten Ventils gegeneinander mit einer Federkraft beaufschlagt. Vorzugsweise ist die Federanordnung dabei in Form einer Knackfroschanordnung oder einer Schnappscheibe ausgebildet, um beim Öffnen und/oder Schließen des ersten Ventils und/oder des zweiten Ventils wiederum ein akustisch detektierbares Signal zu erzeugen. Es ist jedoch auch denkbar, dass die Ventilanordnung selbst kein akustisch detektierbares Signal erzeugt, sondern durch eine gekoppelte Signalerzeugungseinheit ein akustisch oder anderweitig detektierbares Signal erzeugt wird. Beispielsweise können auch andere Federelemente unter Verwendung elastischer fester Elemente oder volumenelastischer Fluide wie etwa Gasdruckfedern eingesetzt werden.

Wenn das erste Ventil und/oder das zweite Ventil als Magnetventile ausgestaltet sind, weisen sie vorzugsweise jeweils einen Magnetring und eine Magnetscheibe sowie ein zwischen dem Magnetring und der Magnetscheibe angeordnetes Dichtelement auf. Das Dichtelement ist dabei vorzugsweise aus einem solchen Material gestaltet, dass das akustische Signal, das die Magnetventile beim Öffnen bzw. Schließen erzeugen, nicht unterdrückt wird. Wenn sowohl das erste Ventil als auch das zweite Ventil ein derartiges Dichtelement aufweisen, kann dieses bei beiden Ventilen aus demselben Material gebildet sein oder aber aus unterschiedlichen Materialien. Beispielsweise können hier Materialien eingesetzt werden, die eine unterschiedliche Abschwächung eines Magnetfelds verursachen. Auf diese Weise ist es möglich, mittels gleich dicker Dichtungselemente eine unterschiedliche Anziehungs- bzw. Abstoßungskraft zwischen der jeweiligen Magnetscheibe und dem jeweiligen Magnetring des ersten Ventils und des zweiten Ventils zu erreichen.

In einer weiteren Variante weisen sowohl das erste Ventil als auch das zweite Ventil jeweils einen Magnetring, eine Magnetscheibe und ein dazwischen angeordnetes Dichtelement auf, wobei das Dichtelement des ersten Ventils eine von der Dicke des Dichtelements des zweiten Ventils abweichende Dicke aufweist. Die Dicke ist dabei die Ausdehnung des Dichtelements entlang einer Achse, die den Magnetring und die Magnetscheibe miteinander verbindet und vorzugsweise senkrecht zu beiden Elementen steht. Vorzugsweise ist die Dicke des Dichtelements des ersten Ventils dabei geringer als die Dicke des Dichtelements des zweiten Ventils. Auf diese Weise ist es möglich, für das erste Dichtelement und das zweite Dichtelement das gleiche Material zu verwenden, aber dennoch eine unterschiedlich starke Anziehungs- bzw. Abstoßungskraft zwischen dem Magnetring und der Magnetscheibe des ersten Ventils im Vergleich zum Magnetring und der Magnetscheibe des zweiten Ventils zu erreichen. Wenn die Magnetscheibe und der Magnetring des ersten und des zweiten Ventils jeweils derart gepolt sind, dass sie einander abstoßen, bewirkt eine geringe Dicke des Dichtelements des ersten Ventils eine stärkere Abstoßungskraft zwischen der Magnetscheibe und dem Magnetring des ersten Ventils im Vergleich zur Magnetscheibe und zum Magnetring des zweiten Ventils. Dadurch wird erreicht, dass das erste Ventil leichter geöffnet werden kann als das zweite Ventil. Aufgrund der Wirkverbindung zwischen dem ersten Ventil und dem zweiten Ventil bedeutet dies, dass das zweite Ventil leichter geschlossen werden kann, als es geöffnet werden kann.

In einer anderen Variante ist die Dicke des Dichtelements des zweiten Ventils geringer als die Dicke des Dichtelements des ersten Ventils. Wenn die Magnetscheibe und der Magnetring des ersten und des zweiten Ventils jeweils derart gepolt sind, dass sie einander anziehen, bewirkt eine geringe Dicke des Dichtelements des zweiten Ventils eine stärkere Anziehungskraft zwischen der Magnetscheibe und dem Magnetring des zweiten Ventils im Vergleich zur Magnetscheibe und zum Magnetring des ersten Ventils. Auch auf diese Weise lassen sich die vorgenannten Effekte erzielen.

Die Wirkverbindung zwischen dem ersten Ventil und dem zweiten Ventil kann - unabhängig von der konkreten Ausgestaltung der Ventile - beispielsweise durch eine Stange realisiert werden, die ein Element des ersten Ventils mit einem Element des zweiten Ventils verbindet. Im Falle von Magnetventilen, die mittels Magnetscheiben und Magnetringen ausgestattet sind, kann die Wirkverbindung beispielsweise durch eine Stange realisiert werden, die die Magnetscheibe des ersten Ventils mit der Magnetscheibe des zweiten Ventils verbindet und dabei durch den Magnetring des ersten Ventils und den Magnetrings des zweiten Ventils hindurchgreift. In dieser Anordnung würde die Stange zudem durch das Dichtelement des ersten Ventils und das Dichtelement des zweiten Ventils hindurchgreifen.

Andere Wirkverbindungen sind aber ebenfalls denkbar. So kann eine Umschaltung des ersten Ventils beispielsweise ein Signal erzeugen, das einen Mechanismus aktiviert, der die Umschaltung des zweiten Ventils bewirkt. Die Wirkverbindung zwischen dem ersten Ventil und dem zweiten Ventil muss also nicht notwendigerweise durch mechanische Mittel realisiert sein; vielmehr sind sämtliche andere Wirkverbindungen wie etwa elektrische oder magnetische Wirkverbindung ebenfalls denkbar.

In einer weiteren Variante weist die Wand ein elastisches Material auf. Insbesondere ist sie vollständig aus dem elastischen Material gebildet. Es ist jedoch auch denkbar, dass die Wand nur abschnittsweise ein elastisches Material aufweist und in anderen Abschnitten unelastisch ausgebildet ist. In einer weiteren Variante ist die Wand nicht elastisch ausgebildet, sondern mit einem elastischen Element verbunden, das eine Beweglichkeit der Wand gegenüber der Gehäusewand ermöglicht, auch wenn die Wand selbst in sich starr ist.

In einer weiteren Variante ist die Wand als Membran oder als Teil eines Kolbens, Ringkolbens, Balgs oder Ringbalgs ausgeführt. Wenn die Wand als Membran ausgeführt ist, besteht sie vorzugsweise aus einem elastischen Material. Dann ist es möglich, dass sich die Wand beim Einströmen eines Fluids in die erste Kammer in Richtung auf die zweite Kammer ausdehnt. Ein Abschnitt der Wand kann dann mit der Ventilanordnung in Wechselwirkung treten, um den Fluiddruck des in die erste Kammer eingeströmten Fluids in eine Kraft umzusetzen, die zur Öffnung des zweiten Ventils eingesetzt wird. Wenn die Wand als Teil eines Kolbens, Ringkolbens, Balgs oder Ringbalgs ausgeführt ist, ist es nicht erforderlich, dass die Wand aus einem elastischen Material besteht oder ein elastisches Material aufweist. Vielmehr kann die Wand starr ausgeführt werden, da ihre Beweglichkeit gegenüber der Gehäusewand durch eine Beweglichkeit des Kolbens oder Balgs gewährleistet wird. In dieser Variante kommt es also im Wesentlichen zu einer Lateralverschiebung der Wand gegenüber der Gehäusewand, und zwar von der ersten Kammer weg auf die zweite Kammer, wenn ein Fluid in die erste Kammer einströmt.

In einer weiteren Variante ist die Wand dazu vorgesehen und eingerichtet, sich zumindest abschnittsweise an ein Betätigungselement anlegen zu können, um auf diese Weise eine Kraft auf das Betätigungselement ausüben zu können. Das Betätigungselement ist dabei mit der Ventilanordnung, insbesondere mit dem ersten Ventil und/oder dem zweiten Ventil, verbunden. Wenn die Wand aus einem starren Material gefertigt ist und lediglich als Teil eines Kolbens oder Balgs lateral verschoben wird, kann ein größerer flächiger Abschnitt zur Anlage an dem Betätigungselement kommen. Wenn die Wand als Membran ausgebildet ist, kommt vorzugsweise nur ein kleinerer Abschnitt der Wand zur Anlage an das Betätigungselement. Für eine entsprechende Kraftübertragung von der Wand auf das Betätigungselement und damit auf die Ventilanordnung ist dies letztlich aber nicht entscheidend.

Das Betätigungselement kann beispielsweise als ringförmige oder scheibenförmige Platte ausgestaltet sein, die unmittelbar mit dem zweiten Ventil, insbesondere mit einer Magnetscheibe des zweiten Ventils, verbunden ist. Wenn dann eine Kraft auf diese Platte durch die Wand ausgeübt wird, kommt es zur unmittelbaren Kraftübertragung auf das zweite Ventil. Auf diese Weise ist es besonders einfach möglich, dass die von der Wand auf das Betätigungselement ausgeübte Kraft zu einer Überführung des zweiten Ventils in seine geöffnete Stellung ausgenutzt werden kann. Dabei sorgt eine Bewegung der Wand also für eine Bewegung des zweiten Ventils in dessen Öffnungsrichtung.

In einer weiteren Variante ist die Vorrichtung dazu vorgesehen und eingerichtet, den Volumenstrom einer Körperflüssigkeit zu messen. Das heißt, als Fluid kommt dabei eine Körperflüssigkeit zum Einsatz. Insbesondere ist vorgesehen, den Volumenstrom von Hirnwasser (Liquor) zu messen. Da die zu messenden Volumina von Liquor und anderen Körperflüssigkeiten regelmäßig geringer sind als in anderen Anwendungen, muss die Vorrichtung in dieser Variante in miniaturisierter Form ausgeführt sein. Zudem sollte sie aus biokompatiblem Material bestehen, um eine Interaktion mit der Körperflüssigkeit auf ein Mindestmaß zu reduzieren oder ganz ausschließen zu können. Wenn der Volumenstrom von Hirnwasser gemessen werden soll, wird der Einlass der Vorrichtung mit einem Ventrikelkatheter verbunden, der mit seiner Spitze in der Hirnkammer des Patienten platziert ist. Der Auslass der Vorrichtung wird in dieser Variante mit einem peripheren Katheter verbunden, der eine Abführung des Hirnwassers in einen Hohlraum des Körpers gestattet. Im erstgenannten Ventrikelkatheter oder im peripheren Katheter ist zudem in üblicher Weise ein weiteres Ventil vorgesehen, das den Rückstrom von Hirnwasser verhindert und eine Überdruckfunktion hat.

In einer weiteren Variante ist die Vorrichtung dazu vorgesehen und eingerichtet, unter der Haut eines Patienten implantiert zu werden. Auch in dieser Variante sollte die Vorrichtung möglichst vollständig aus einem biokompatiblen Material bestehen, um weder eine Interaktion mit der Körperflüssigkeit, deren Volumenstrom gemessen werden soll, noch eine Interaktion mit dem Körper des Patienten einzugehen, die über die reine Messung des Volumenstroms der Körperflüssigkeit hinausgeht. Bei einer vorgesehenen Implantation der Vorrichtung ist insbesondere eine Variante von Vorteil, bei der ein akustisch detektierbares Signal von der Ventilanordnung erzeugt wird. Dieses akustisch detektierbare Signal lässt sich dann auf besonders einfache Weise und außerhalb des Körpers des Patienten mittels eines Mikrofons oder eines Stethoskops detektieren.

In einer weiteren Variante sind das erste Ventil und das zweite Ventil derart ausgelegt, dass eine Überführung von ihrer geschlossenen Stellung in ihre geöffnete Stellung und umgekehrt sprungartig erfolgt. Mit anderen Worten ausgedrückt, sind die Ventile in dieser Variante derart ausgebildet, dass eine Schaltzeit, die die Ventile zur Überführung von ihrer geöffneten Position in ihre geschlossene Position benötigen, signifikant kleiner ist als die Zeit, in der sich die Ventile entweder in der vollständig geöffneten oder der vollständig geschlossenen Stellung befinden. Vorzugsweise beträgt die Schaltzeit lediglich 1/10, insbesondere 5/100, insbesondere 1/100 und ganz besonders 5/1000 des Zeitraums, in dem die Ventile in ihrer vollständig geöffneten Stellung oder ihrer vollständig geschlossenen Stellung vorliegen. In absoluten Werten ausgedrückt, beträgt die Schaltzeit der Ventile vorzugsweise 0,5 s oder weniger, insbesondere 0,3 s oder weniger, insbesondere 0,1 s oder weniger, insbesondere 0,05 s oder weniger und ganz besonders 0,01 s oder weniger. Auf diese Weise kann gewährleistet werden, dass die Kraft-Weg-Kennlinie der Ventile sowohl bei der Überführung der Ventilanordnung von der ersten Stellung (stabile Stellung, in der das erste Ventil geöffnet und das zweite Ventil geschlossen ist) in die zweite Stellung (metastabile Stellung, in der erste Ventil geschlossen und das zweite Ventil geöffnet ist) jeweils einen sprungartigen Verlauf aufweist, wobei sich an einen Abschnitt, bei dem bei sinkender auf die Ventilanordnung wirkender Kraft zunächst keine Änderung der relativen Position der einzelnen Elemente der Ventilanordnung eintritt und dann bei einer bestimmten Übergangskraft ohne signifikante Kraftänderung eine Überführung der Ventilanordnung von der einen in die andere Stellung erfolgt, woraufhin eine anschließende Änderung der Kraft wiederum keinen signifikanten Einfluss auf die relative Position der einzelnen Elemente der Ventilanordnung hat. Ein solcher stufenartiger bzw. sprungartiger Verlauf der Kraft-Weg-Kennlinie ist indikativ für ein sprungartiges Überführen der Ventilanordnung von der stabilen Stellung in die metastabile Stellung und umgekehrt.

Durch eine derartige Verkürzung der Schaltzeit kann sichergestellt werden, dass pro Umschaltvorgang der Ventilanordnung jeweils nur ein sehr geringes Volumen des Fluids von der ersten Kammer in die zweite Kammer übertritt. Zudem kann auf diese Weise sichergestellt werden, dass dieses Volumen sehr genau bestimmt werden kann, da ein Übertreten des Fluids von der ersten Kammer in die zweite Kammer während des Schaltvorgangs selbst im Wesentlichen nicht möglich ist, da dieser Schaltvorgang zu schnell erfolgt. Somit gewährleistet die Realisierung des Schaltvorgangs in einer kurzen Zeitspanne eine hohe Genauigkeit der Vorrichtung.

Die Erfindung betrifft auch eine Anordnung (ein System) zur Messung eines Volumenstroms eines in einer Leitung strömenden Fluids mit den nachfolgend erläuterten Merkmalen. Zunächst weist eine solche Anordnung eine Vorrichtung gemäß den obigen Erläuterungen auf, wobei ein akustisch detektierbares Signal erzeugt wird, wenn das erste Ventil und/oder das zweite Ventil geöffnet und/oder geschlossen werden. Weitere Varianten der oben erläuterten Vorrichtung sind ebenfalls möglich und mit der Variante der Erzeugung eines akustisch detektierbaren Signals in beliebiger Weise kombinierbar. Ferner weist die Anordnung ein Mikrofon auf, das in einem solchen Abstand von der Vorrichtung angeordnet ist, dass es ein von der Vorrichtung erzeugtes akustisches Signal detektieren kann. Schließlich ist die Anordnung noch mit einer Auswerteeinheit ausgestattet, die in Wirkverbindung mit dem Mikrofon steht. Die Auswerteeinheit ist dazu vorgesehen und eingerichtet, ein von dem Mikrofon erzeugtes elektrisches Signal in einen Volumenstrom umzurechnen. Damit eignet sich eine solche Anordnung dazu, auf der Basis eines akustisch detektierbaren Signals eine einfach durchzuführende und sehr genaue Volumenstrommessung eines strömenden Fluids vornehmen zu können.

In einer Variante umfasst die Auswerteeinheit der Anordnung eine Schallerkennungseinheit, eine Zähleinheit und eine Recheneinheit. Als Schallerkennungseinheit kann beispielsweise ein Analog-Digital-Wandler eingesetzt werden, der ein von dem Mikrofon erzeugtes analoges Signal in ein digitales Signal umwandelt. Dabei kann zusätzlich etwaiges Rauschen unterdrückt werden, so dass ein "echtes" akustisches Signal, das von der Vorrichtung durch Öffnen und/oder Schließen eines der beiden Ventile der Ventilanordnung erzeugt wird, von Artefakten unterschieden werden kann. Die Zähleinheit ist dazu vorgesehen und eingerichtet, die von der Schallerkennungseinheit bereitgestellten Signale zu zählen und eine Summe der gezählten Signale zu erzeugen. Die Recheneinheit dient schließlich dazu, auf der Grundlage der Summe der gezählten Signale einen Volumenstrom des strömenden Fluids zu berechnen. Dazu kann die Recheneinheit auf Daten zugreifen, die eine Korrelation zwischen Signalen (die jeweils einem Umschaltvorgang der Ventilanordnung entsprechen) und einem Volumen herzustellen, das pro Umschaltvorgang von der ersten Kammer in die zweite Kammer strömt. Schließlich ist die Zähleinheit und/oder die Recheneinheit noch mit einem Zeit- oder Taktgeber versehen, so dass die gezählten Signale in Korrelation mit einer Zeiteinheit gesetzt werden können. Auf diese Weise lässt sich dann als Ergebnis ein Volumenstrom (Volumen pro Zeiteinheit) des durch die Vorrichtung strömenden Fluids angeben.

Die Erfindung betrifft schließlich auch ein Verfahren zur Messung eines Volumenstroms eines in einer Leitung strömenden Fluids unter Verwendung einer Vorrichtung gemäß den obigen Erläuterungen. Selbstverständlich kann statt einer Vorrichtung auch eine Anordnung entsprechend den vorherigen Erläuterungen eingesetzt werden.

Dieses Verfahren zeichnet sich durch die nachfolgend erläuterten Schritte aus. Zunächst wird eine Vielzahl von Signalen detektiert, die von der Vorrichtung bei jedem Schließen und/oder Öffnen eines ersten Ventils und/oder eines zweiten Ventils erzeugt wird. Anschließend erfolgt eine Bestimmung der Anzahl der detektierten Signale pro Zeiteinheit. Schließlich wird der Volumenstrom des Fluids auf der Grundlage der Anzahl der detektierten Signale pro Zeiteinheit und auf der Grundlage von Daten, die angeben, welches Volumen eines die Vorrichtung durchströmenden Fluids im Zeitraum zwischen Öffnen des ersten Ventils und/oder des zweiten Ventils und Schließen des ersten Ventils und/oder des zweiten Ventils durch die Vorrichtung strömt. Diese Art der Berechnung wurde weiter oben bereits hinlänglich erläutert, so dass zur Vermeidung von Wiederholungen auf die obigen Erläuterungen verwiesen wird.

Die oben erläuterten alternativen Ausgestaltungen der Vorrichtung sind in analoger Weise auch auf die beanspruchte Anordnung und das beanspruchte Verfahren übertragbar, und umgekehrt. Ferner sind sämtliche der oben erläuterten alternativen Ausgestaltungen in beliebiger Weise kombinierbar, sofern dies technisch machbar ist.

Weitere Einzelheiten der vorliegenden Erfindung werden anhand eines Ausführungsbeispiels und entsprechender Figuren nachfolgend erläutert. Es zeigen:
- Figur 1: ein Ausführungsbeispiel einer Vorrichtung zur Messung eines Volumenstroms in einer ersten Stellung und
- Figur 2: das Ausführungsbeispiel der Figur 1 in einer zweiten Stellung.

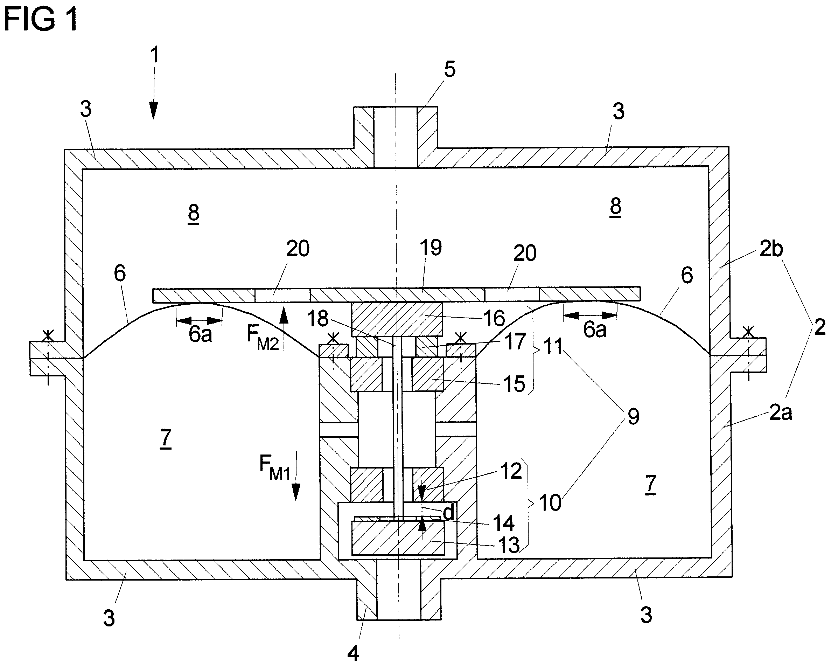

Die Figur 1 zeigt einen Volumenstrommesser 1 als Vorrichtung zur Messung eines Volumenstroms. Dieser Volumenstrommesser 1 ist aus einem Gehäuse 2 gebildet, das aus einer ersten Gehäusehälfte 2a und aus einer zweiten Gehäusehälfte 2b besteht. Beide Gehäusehälften 2a, 2b bilden zusammen eine Gehäusewand 3. In der ersten Gehäusehälfte 2a ist die Gehäusewand 3 durch einen Einlassstutzen 4 als Einlass unterbrochen. Dem Einlassstutzen 4 direkt gegenüber liegt in der zweiten Gehäusehälfte 2b ein Auslassstutzen 5, der wiederum eine Unterbrechung der Gehäusewand 3 bildet und als Auslass dient.

Zwischen der ersten Gehäusehälfte 2a und der zweiten Gehäusehälfte 2b ist eine Membran 6 eingespannt, die als fluiddichte Wand dient, die gegenüber der Gehäusewand 3 beweglich ist. Die Membran 6 ist ringförmig ausgestaltet und besteht aus einem elastischen Material. Durch die Membran 6 wird der Innenraum des Gehäuses 2 in eine erste Kammer 7 und in eine zweite Kammer 8 unterteilt. Ein Fluid, das sich in der ersten Kammer 7 befindet, kann nicht durch die Membran 6 hindurch in die zweite Kammer 8 gelangen, da die Membran 6 fluiddicht ist. Gleichfalls kann ein Fluid, das sich in der zweiten Kammer 8 befindet, nicht durch die Membran 6 hindurch in die erste Kammer 7 gelangen.

Im Innenraum des Gehäuses 2 ist zudem eine Ventilanordnung 9 vorgesehen, die aus einem ersten Ventil 10 und einem zweiten Ventil 11 besteht. Das erste Ventil 10 und das zweite Ventil 11 sind als Magnetventile ausgestaltet. Das erste Ventil 10 ist aus einem ersten Magnetring 12, einer ersten Magnetscheibe 13 und einem zwischen dem ersten Magnetring 12 und der ersten Magnetscheibe 13 angeordneten ersten Dichtring 14, der als Dichtelement dient, aufgebaut. Dabei ist die erste Magnetscheibe 13 dem Einlassstutzen 4 zugewandt, während der erste Magnetring 12 der ersten Kammer 7 zugewandt ist.

Das zweite Ventil 11 ist in analoger Weise aus einem zweiten Magnetring 15, einer zweiten Magnetscheibe 16 und einem zwischen dem zweiten Magnetring 15 und der zweiten Magnetscheibe 16 angeordneten zweiten Dichtring 17, der wiederum als Dichtelement dient, aufgebaut. Der zweite Magnetring 15 ist dabei zur ersten Kammer 7 hin orientiert, während die zweite Magnetscheibe 16 zur zweiten Kammer 8 hin orientiert ist.

Damit ist das erste Ventil 10 zwischen dem Einlassstutzen 4 und der ersten Kammer 7 angeordnet, während das zweite Ventil 11 zwischen der ersten Kammer 7 und der zweiten Kammer 8 angeordnet ist. Ein Fluid, das den Volumenstrommesser 1 durchströmt, muss zuerst das erste Ventil 10 passieren, um in die erste Kammer 7 einzutreten. Von dort kann das Fluid dann durch das zweite Ventil 11 hindurch in die zweite Kammer eintreten, um den Volumenstrommesser 1 schließlich durch den Auslassstutzen 5 wieder zu verlassen.

Die erste Magnetscheibe 13 und die zweite Magnetscheibe 16 sind miteinander durch eine Stange 18 verbunden, die eine Wirkverbindung zwischen dem ersten Ventil 10 und dem zweiten Ventil 11 herstellt.

Der erste Magnetring 12 und die erste Magnetscheibe 13 sind derart gepolt, dass sie einander abstoßen. Ebenso sind der zweite Magnetring 15 und die zweite Magnetscheibe 16 derart gepolt, dass sie einander abstoßen. Der erste Magnetring 12 und der zweite Magnetring 15 sind fest mit der Gehäusewand 3 des Gehäuses 2 verbunden. Demgegenüber sind die erste Magnetscheibe 13 und die zweite Magnetscheibe 16 beweglich gelagert. Auf die erste Magnetscheibe 13 wird durch den ersten Magnetring 12 eine Abstoßungskraft in Richtung des mit F_{M1} gekennzeichneten Pfeil ausgeübt. In analoger Weise wird auf die zweite Magnetscheibe 16 durch den zweiten Magnetring 15 eine Abstoßungskraft in Richtung des mit F_{M2} gekennzeichneten Pfeils ausgeübt. Die Abstoßungskraft F_{M1} ist dabei größer als die Abstoßungskraft F_{M2}, da der erste Dichtring 14 eine geringere Dicke aufweist als der zweite Dichtring 17. Folglich liegt die Ventilanordnung 9 immer dann, wenn keine externe Kraft auf sie ausgeübt wird, in einer ersten Stellung vor, in der das erste Ventil 10 geöffnet und das zweite Ventil 11 geschlossen ist.

In einer anderen, hier nicht dargestellten Ausführungsform ziehen sich die Magnetscheibe und der Magnetring eines jeden Ventils an. Dann ist der erste Dichtring dicker ausgeführt als der zweite Dichtring, um zu erreichen, dass das zweite Ventil in seiner geschlossenen Stellung vorliegt, wenn keine externe Kraft in Öffnungsrichtung des zweiten Ventils aufgebracht wird.

Wenn die Membran 6 nun eine Kraft in Öffnungsrichtung des zweiten Ventils 11 (diese Richtung entspricht der Richtung der Abstoßungskraft F_{M2}) auf das zweite Ventil aufbringt, kann die Ventilanordnung 9 von ihrer ersten stabilen Stellung in eine zweite, metastabile Stellung überführt werden, in der das erste Ventil 10 geschlossen und das Ventil 11 geöffnet ist.

In der Darstellung der Figur 1 befindet sich das erste Ventil 10 in seiner geöffneten Stellung und das zweite Ventil 11 in seiner geschlossenen Stellung. Die Ventilanordnung 9 befindet sich also in der stabilen Stellung. Die erste Magnetscheibe 13 und der erste Dichtring 14 sind in dieser Stellung in einem Abstand d voneinander beabstandet angeordnet. In dieser Stellung kann ein Fluid durch den Einlassstutzen 4 und das erste Ventil 10 in die erste Kammer 7 eintreten und die Membran 6 in Richtung auf die zweite Kammer 8 ausdehnen. Ein Übertritt des Fluids in die zweite Kammer 8 ist indes nicht möglich, da das zweite Ventil 11 geschlossen ist.

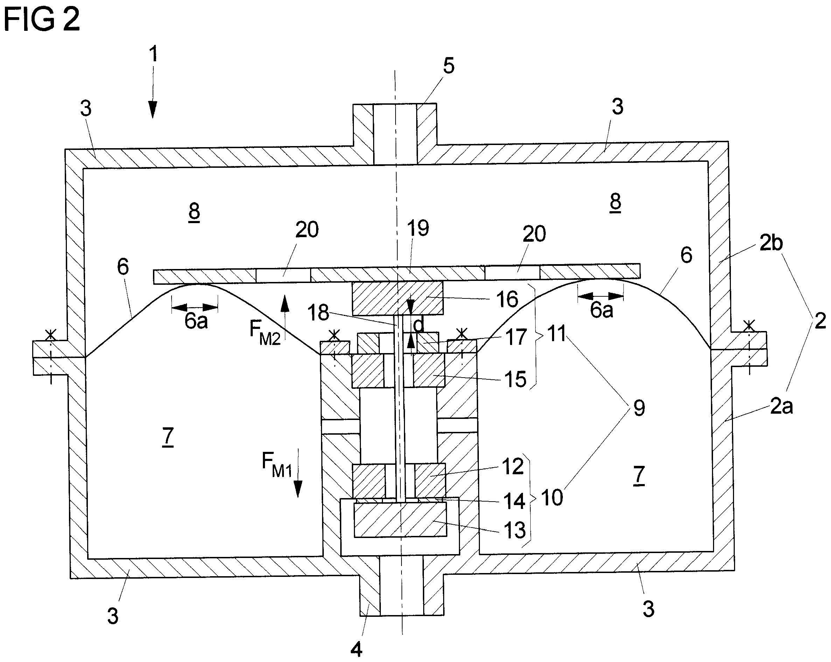

Die Figur 2 zeigt den Volumenstrommesser 1 der Figur 1 mit der Ventilanordnung 9 in der metastabilen Position. Gleiche Elemente werden mit gleichen Bezugszeichen wie in der Figur 1 versehen; es wird auf die diesbezüglichen Ausführungen zur Figur 1 verwiesen.

In dieser metastabilen Stellung der Ventilanordnung 9 ist das erste Ventil 10 geschlossen und das zweite Ventil 11 geöffnet. Die Überführung der Ventilanordnung 9 von der in der Figur 1 dargestellten stabilen Position in die in der Figur 2 dargestellte metastabile Position erfolgt im Laufe des Einströmens von Fluid in die erste Kammer 7. Denn wie bereits erwähnt, dehnt sich die Membran 6 dabei in Richtung auf die zweite Kammer aus. Dabei kontaktiert die Membran 6 eine ringförmige Scheibe 19, die mit der zweiten Magnetscheibe 16 verbunden ist. Der Kontakt zwischen der Membran 6 und der Scheibe 19 erfolgt dabei nicht über die gesamte Fläche der Membran 6, sondern nur in einem Abschnitt 6a der Membran 6. Wenn die von der Membran 6 infolge des von dem Fluid in der ersten Kammer 7 aufgebauten Drucks auf die Scheibe 19 übertragene Kraft größer ist als der Betrag der Differenz aus den Kräften F_{M1} - F_{M2}, verbleibt eine resultierende Kraft in Öffnungsrichtung des zweiten Ventils 11. Dadurch wird die Scheibe 19 in Richtung auf den Auslassstutzen 5 bewegt und bewegt damit ihrerseits die gesamte Ventilanordnung 9 in Richtung auf den Auslassstutzen 5. Dadurch wird das erste Ventil 10 geschlossen und das zweite Ventil 11 geöffnet. Die zweite Magnetscheibe 16 ist nun in einem Abstand d zu dem zweiten Dichtring 17 angeordnet, der identisch ist mit dem Abstand d, der zwischen der ersten Magnetscheibe 13 und dem ersten Dichtring 14 in der stabilen Position der Ventilanordnung 9 ausgebildet ist.

Die Scheibe 19 ist mit Öffnungen 20 versehen, durch die hindurch das Fluid ungehindert strömen kann, um zum Auslassstutzen 5 zu gelangen.

Wenn das zweite Ventil 11 geöffnet ist, kann das in der ersten Kammer 7 befindliche Fluid durch das zweite Ventil 11 hindurch in die zweite Kammer 8 eintreten. Da sich die Wand 6 dabei in Richtung auf die erste Kammer 7 bewegt, vergrößert sich das dem Fluid in der zweiten Kammer 8 zur Verfügung stehende Volumen. Folglich kommt es in dieser Ventilstellung noch nicht zu einem Fluidaustritt aus dem Volumenstrommesser 1. Wenn sich das Volumen des Fluids in der ersten Kammer 7 etwas verringert hat, genügt die vom Fluiddruck in der ersten Kammer 7 aufgebaute und auf die Membran 6 wirkende Kraft nicht mehr aus, um die Ventilanordnung 9 in ihrer metastabilen Stellung zu halten. Vielmehr springt die Ventilanordnung 9 in ihre stabile Position zurück, in der das erste Ventil 10 geöffnet und das zweite Ventil 11 geschlossen ist. Nun kann neues Fluid durch das erste Ventil 10 hindurch in die erste Kammer 7 einströmen und erneut einen Fluiddruck in der ersten Kammer 7 aufbauen, der in eine entsprechende auf die Ventilanordnung 9 wirkende Kraft umgesetzt wird. In dieser Phase und tritt ein Teil des in der zweiten Kammer 8 befindlichen Fluids durch den Auslassstutzen 5 aus der zweiten Kammer 8 und damit aus dem Volumenstrommesser 1 aus. Denn durch die Bewegung der Wand 6 in Richtung auf die zweite Kammer 8 verringert sich das Volumen, das dem Fluid in der zweiten Kammer 8 zur Verfügung steht. Anschließend springt die Ventilanordnung 9 wieder in ihre metastabile Position, in der das Fluid aus der ersten Kammer 7 in die zweite Kammer 8 strömen kann.

Während des Betriebs des Volumenstrommessers 1 kommt es zu sehr häufigen Umschaltvorgängen der Ventilanordnung 9 von der stabilen Position in die metastabile Position und wieder zurück in die stabile Position. Das Fluidvolumen, das immer dann, wenn die Ventilanordnung in ihrer metastabilen Stellung vorliegt, aus der ersten Kammer 7 in die zweite Kammer 8 übertreten kann, lässt sich auf einfache Weise vorbestimmen oder ermitteln. Folglich kann dann durch Zählen der Umschaltvorgänge der Ventilanordnung 9 pro Zeiteinheit bestimmt werden, welches Fluidvolumen durch den Volumenstrommesser 1 hindurch strömt.

## Patentansprüche

1. Vorrichtung zur Messung eines Volumenstroms eines in einer Leitung strömenden Fluids, mit einem durch eine Gehäusewand (3) gebildeten Gehäuse (2), einem Einlass (4), durch den hindurch ein Fluid aus einem ersten Abschnitt einer Leitung in das Gehäuse (2) eintreten kann, einem Auslass (5), durch den ein Fluid aus dem Gehäuse (2) austreten kann, und einer gegenüber der Gehäusewand (3) zumindest abschnittsweise beweglichen und fluiddichten Wand (6), die das Gehäuse (2) in eine erste Kammer (7) und in eine zweite Kammer (8) teilt, wobei in dem Gehäuse (2) ein erstes Ventil (10) und ein zweites Ventil (11) angeordnet sind, die derart miteinander in Wechselwirkung stehen, dass eine Überführung des ersten Ventils (10) oder des zweiten Ventils (11) von seiner geöffneten Stellung in seine geschlossene Stellung eine gleichzeitige Überführung des jeweils anderen Ventils (11, 10) von seiner geschlossenen Stellung in seine geöffnete Stellung bewirkt und dass eine Überführung des ersten Ventils (10) oder des zweiten Ventils (11) von seiner geschlossenen Stellung in seine geöffnete Stellung eine gleichzeitige Überführung des jeweils anderen Ventils (11, 10) von seiner geöffneten Stellung in seine geschlossene Stellung bewirkt, wobei die Wand (6) mit dem ersten Ventil (10) und dem zweiten Ventil (11) in Wechselwirkung steht, so dass eine Kraft, die zum Öffnen des zweiten Ventils (11) benötigt wird, auf das erste Ventil (10) und das zweite Ventil (11) mittels einer Bewegung zumindest eines Abschnitts (6a) der Wand (6) übertragen werden kann,
**dadurch gekennzeichnet,**
**dass** das erste Ventil (10) zwischen dem Einlass (4) und der ersten Kammer (7) angeordnet ist und dass das zweite Ventil (11) zwischen der ersten Kammer (7) und der zweiten Kammer (8) angeordnet ist,
**dass** ein Schließen und/oder Öffnen des ersten Ventils (10) und/oder des zweiten Ventils (11) ein detektierbares Signal erzeugt und
**dass** die Kraft, die zum Öffnen des zweiten Ventils (11) benötigt wird, größer ist als eine Kraft, die zum Schließen des zweiten Ventils (11) benötigt wird, so dass die Ventilanordnung in einer stabilen Stellung, in der das erste Ventil geöffnet und das zweite Ventil geschlossen ist, und in einer metastabilen Stellung, in der das in der das erste Ventil geschlossen und das zweite Ventil geöffnet ist, vorliegen kann.

2. Vorrichtung nach Anspruch 1, **dadurch gekennzeichnet, dass** das beim Öffnen und/oder Schließen des ersten Ventils (10) und/oder zweiten Ventils (11) erzeugte Signal ein akustisch detektierbares Signal ist.

3. Vorrichtung nach Anspruch 1 oder 2, **dadurch gekennzeichnet, dass** das erste Ventil (10) und/oder das zweite Ventil (11) als Magnetventile ausgeführt sind.

4. Vorrichtung nach Anspruch 3, **dadurch gekennzeichnet, dass** das erste Ventil (10) und/oder das zweite Ventil (11) jeweils einen Magnetring (12, 15) und eine Magnetscheibe (13, 16) sowie ein zwischen dem Magnetring (12, 15) und der Magnetscheibe (13, 16) angeordnetes Dichtelement (14, 17) aufweisen.

5. Vorrichtung nach Anspruch 3 oder 4, **dadurch gekennzeichnet, dass** das erste Ventil (10) und das zweite Ventil (11)jeweils einen Magnetring (12, 15) und eine Magnetscheibe (13, 16) sowie ein zwischen dem Magnetring (12, 15) und der Magnetscheibe (13, 16) angeordnetes Dichtelement (14, 17) aufweisen, wobei das Dichtelement (14) des ersten Ventils (10) eine geringere Dicke als das Dichtelement (17) des zweiten Ventils (11) aufweist.

6. Vorrichtung nach einem der vorhergehenden Ansprüche, **dadurch gekennzeichnet, dass** die Wand (6) ein elastisches Material aufweist.

7. Vorrichtung nach einem der vorhergehenden Ansprüche, **dadurch gekennzeichnet, dass** die Wand (6) als Membran oder als Teil eines Kolbens, Ringkolbens, Balgs oder Ringbalgs ausgeführt ist.

8. Vorrichtung nach einem der vorhergehenden Ansprüche, **dadurch gekennzeichnet, dass** die Wand (6) dazu vorgesehen und eingerichtet ist, sich zumindest abschnittsweise an ein Betätigungselement (19) anlegen zu können, um auf diese Weise eine Kraft auf das Betätigungselement (19) ausüben zu können, wobei das Betätigungselement (19) mit dem ersten Ventil (10) und/oder dem zweiten Ventil (11) verbunden ist.

9. Vorrichtung nach einem der vorhergehenden Ansprüche, **dadurch gekennzeichnet, dass** die Vorrichtung dazu vorgesehen und eingerichtet ist, den Volumenstrom einer Körperflüssigkeit zu messen.

10. Vorrichtung nach einem der vorhergehenden Ansprüche, **dadurch gekennzeichnet, dass** die Vorrichtung dazu vorgesehen und eingerichtet ist, unter der Haut eines Patienten implantiert zu werden.

11. Vorrichtung nach einem der vorhergehenden Ansprüche, **dadurch gekennzeichnet, dass** das erste Ventil (10) und das zweite Ventil (11) derart ausgelegt sind, dass eine Überführung von ihrer geschlossenen Stellung in ihre geöffnete Stellung und umgekehrt sprungartig erfolgt.

12. Anordnung zur Messung eines Volumenstroms eines in einer Leitung strömenden Fluids, **gekennzeichnet durch**
• eine Vorrichtung (1) nach Anspruch 2 oder einem der Ansprüche 3 bis 11, soweit rückbezogen auf Anspruch 2,
• ein Mikrofon, das in einem solchen Abstand von der Vorrichtung (1) angeordnet ist, dass es ein von der Vorrichtung (1) erzeugtes akustisches Signal detektieren kann,
• eine in Wirkverbindung mit dem Mikrofon stehende Auswerteeinheit, die dazu vorgesehen und eingerichtet ist, ein von dem Mikrofon erzeugtes elektrisches Signal in einen Volumenstrom umzurechnen.

13. Anordnung nach Anspruch 12, **dadurch gekennzeichnet, dass** die Auswerteeinheit eine Schallerkennungseinheit, eine Zähleinheit und eine Recheneinheit umfasst.

14. Verfahren zur Messung eines Volumenstroms eines in einer Leitung strömenden Fluids unter Verwendung einer Vorrichtung nach einem der Ansprüche 1 bis 11, **gekennzeichnet durch** die folgenden Schritte:
• Detektieren einer Vielzahl von Signalen, die von der Vorrichtung (1) bei jedem Schließen und/oder Öffnen eines ersten Ventils (10) und/oder eines zweiten Ventils (11) erzeugt wird,
• Bestimmen der Anzahl der detektierten Signale pro Zeiteinheit und
• Berechnen des Volumenstroms auf der Grundlage der Anzahl der detektierten Signale pro Zeiteinheit und auf der Grundlage von Daten, die angeben, welches Volumen eines die Vorrichtung (1) durchströmenden Fluids im Zeitraum zwischen Öffnen des ersten Ventils (10) und/oder zweiten Ventils (11) und Schließen des ersten Ventils (10) und/oder zweiten Ventils (11) **durch** die Vorrichtung (1) strömt.

## Claims

1. Device for measuring a volume flow of a fluid flowing in a line, having a housing (2) which is formed by a housing wall (3), having an inlet (4) through which a fluid can enter the housing (2) from a first section of a line, having an outlet (5) through which a fluid can exit the housing (2), and having a wall (6) which is movable at least in sections in relation to the housing wall (3) and is fluid-tight and which divides the housing (2) into a first chamber (7) and into a second chamber (8), wherein, in the housing (2), there are arranged a first valve (10) and a second valve (11) which interact with one another such that a switching of the first valve (10) or of the second valve (11) from its open position into its closed position brings about a simultaneous switching of the in each case other valve (11, 10) from its closed position into its open position, and such that a switching of the first valve (10) or of the second valve (11) from its closed position into its open position brings about a simultaneous switching of the in each case other valve (11, 10) from its open position into its closed position, wherein the wall (6) interacts with the first valve (10) and with the second valve (11) such that a force which is required for opening the second valve (11) can be transmitted to the first valve (10) and to the second valve (11) by means of a movement of at least one section (6a) of the wall (6),
**characterized**
**in that** the first valve (10) is arranged between the inlet (4) and the first chamber (7), and in that the second valve (11) is arranged between the first chamber (7) and the second chamber (8),
**in that** a closing and/or opening of the first valve (10) and/or of the second valve (11) generates a detectable signal, and
**in that** the force which is required for opening the second valve (11) is larger than a force which is required for closing the second valve (11), with the result that the valve arrangement can be present in a stable position, in which the first valve is open and the second valve is closed, and in a metastable position, in which the first valve is closed and the second valve is open.

2. Device according to Claim 1, **characterized in that** the signal which is generated with the opening and/or closing of the first valve (10) and/or second valve (11) is an acoustically detectable signal.

3. Device according to Claim 1 or 2, **characterized in that** the first valve (10) and/or the second valve (11) are designed as solenoid valves.

4. Device according to Claim 3, **characterized in that** the first valve (10) and/or the second valve (11) have in each case one magnet ring (12, 15) and one magnet disc (13, 16) and also one sealing element (14, 17) which is arranged between the magnet ring (12, 15) and the magnet disc (13, 16).

5. Device according to Claim 3 or 4, **characterized in that** the first valve (10) and the second valve (11) have in each case one magnet ring (12, 15) and one magnet disc (13, 16) and also one sealing element (14, 17) which is arranged between the magnet ring (12, 15) and the magnet disc (13, 16), wherein the sealing element (14) of the first valve (10) has a smaller thickness than the sealing element (17) of the second valve (11).

6. Device according to any of the preceding claims, **characterized in that** the wall (6) comprises an elastic material.

7. Device according to any of the preceding claims, **characterized in that** the wall (6) is designed as a diaphragm or as part of a piston, ring-shaped piston, bellows or ring-shaped bellows.

8. Device according to any of the preceding claims, **characterized in that** the wall (6) is provided and set up to be able to bear at least in sections against an actuation element (19) in order, in this way, to be able to exert a force on the actuation element (19), wherein the actuation element (19) is connected to the first valve (10) and/or to the second valve (11).

9. Device according to any of the preceding claims, **characterized in that** the device is provided and set up to measure the volume flow of a bodily fluid.

10. Device according to any of the preceding claims, **characterized in that** the device is provided and set up to be implanted under the skin of a patient.

11. Device according to any of the preceding claims, **characterized in that** the first valve (10) and the second valve (11) are designed such that they are switched from their closed position into their open position, and vice versa, in an abrupt manner.

12. Arrangement for measuring a volume flow of a fluid flowing in a line, **characterized by**
• a device (1) according to Claim 2 or any of Claims 3 to 11, where referring back to Claim 2,
• a microphone which is arranged at such a distance from the device (1) that it can detect an acoustic signal generated by the device (1),
• an evaluation unit which is operatively connected to the microphone and which is provided and set up to convert an electrical signal, generated by the microphone, into a volume flow.

13. Arrangement according to Claim 12, **characterized in that** the evaluation unit comprises a sound detection unit, a counting unit and a computation unit.

14. Method for measuring a volume flow of a fluid flowing in a line using a device according to any of Claims 1 to 11, **characterized by** the following steps:
• detecting a multiplicity of signals which are generated by the device (1) with each closing and/or opening of a first valve (10) and/or of a second valve (11),
• determining the number of detected signals per unit time, and
• computing the volume flow on the basis of the number of detected signals per unit time, and on the basis of data which specify which volume of a fluid flowing through the device (1) flows through the device (1) over the time period between an opening of the first valve (10) and/or second valve (11) and a closing of the first valve (10) and/or second valve (11).

## Revendications

1. Dispositif de mesure du débit volumique d'un fluide qui s'écoule dans un conduit, l'ensemble présentant
un boîtier (2) formé par une paroi de boîtier (3),
une admission (4) par laquelle un fluide peut pénétrer dans le boîtier (2) en provenance d'une première section d'un conduit,
une sortie (5) par laquelle un fluide peut sortir du boîtier (2) et
une paroi (6) étanche aux fluides et dont au moins certaines parties sont mobiles par rapport à la paroi (3) du boîtier et divisant le boîtier (2) en une première chambre (7) et une deuxième chambre (8),
une première soupape (10) et une deuxième soupape (11) qui coopèrent l'une avec l'autre de telle sorte que le passage de la première soupape (10) ou de la deuxième soupape (11) de sa position ouverte à sa position fermée entraîne un transfert simultané de l'autre soupape (11, 10) de sa position fermée à sa position ouverte et qu'un passage de la première soupape (10) ou de la deuxième soupape (11) de sa position fermée à sa position ouverte entraîne un passage simultané de l'autre soupape (11, 10) de sa position ouverte à sa position fermée étant disposées dans le boîtier (2),
la paroi (6) coopérant avec la première soupape (10) et la deuxième soupape (11) de telle sorte que la force nécessaire pour l'ouverture de la deuxième soupape (11) puisse être transférée sur la première soupape (10) et la deuxième soupape (11) au moyen du déplacement d'au moins une section (6a) de la paroi (6),
**caractérisé en ce que**
la première soupape (10) est disposée entre l'admission (4) et la première chambre (7) et **en ce que** la deuxième soupape (11) est disposée entre la première chambre (7) et la deuxième chambre (8),
**en ce que** la fermeture et/ou l'ouverture de la première soupape (10) et/ou de la deuxième soupape (11) produit un signal détectable et
**en ce que** la force nécessaire pour l'ouverture de la deuxième soupape (11) est plus grande que la force nécessaire pour la fermeture de la deuxième soupape (11), de telle sorte que l'ensemble de soupape puisse se trouver en une position stable dans laquelle la première soupape est ouverte et la deuxième soupape est fermée et en une position métastable dans laquelle la première soupape est fermée et la deuxième soupape est ouverte.

2. Dispositif selon la revendication 1, **caractérisé en ce que** le signal produit lors de l'ouverture et/ou de la fermeture de la première soupape (10) et/ou de la deuxième soupape (11) est un signal détectable acoustiquement.

3. Dispositif selon les revendications 1 ou 2, **caractérisé en ce que** la première soupape (10) et/ou la deuxième soupape (11) sont réalisées sous la forme de soupapes magnétiques.

4. Dispositif selon la revendication 3, **caractérisé en ce que** la première soupape (10) et/ou la deuxième soupape (11) présentent chacune une bague magnétique (12, 15) et un disque magnétique (13, 16) ainsi qu'un élément d'étanchéité (14, 17) disposé entre la bague magnétique (12, 15) et le disque magnétique (13, 16).

5. Dispositif selon les revendications 3 ou 4, **caractérisé en ce que** la première soupape (10) et la deuxième soupape (11) présentent chacune une bague magnétique (12, 15) et un disque magnétique (13, 16) ainsi qu'un élément d'étanchéité (14, 17) disposé entre la bague magnétique (12, 15) et le disque magnétique (13, 16), l'élément d'étanchéité (14) de la première soupape (10) ayant une épaisseur plus petite que l'élément d'étanchéité (17) de la deuxième soupape (11).

6. Dispositif selon l'une quelconque des revendications précédentes, **caractérisé en ce que** la paroi (6) présente un matériau élastique.

7. Dispositif selon l'une quelconque des revendications précédentes, **caractérisé en ce que** la paroi (6) est réalisée sous la forme d'une membrane ou d'une partie d'un piston, d'un piston annulaire, d'un soufflet ou d'un soufflet annulaire.

8. Dispositif selon l'une quelconque des revendications précédentes, **caractérisé en ce que** la paroi (6) est prévue et conçue pour pouvoir se placer au moins en partie contre un élément d'actionnement (19) pour de cette manière pouvoir exercer une force sur l'élément d'actionnement (19), l'élément d'actionnement (19) étant relié à la première soupape (10) et/ou à la deuxième soupape (11).

9. Dispositif selon l'une quelconque des revendications précédentes, **caractérisé en ce que** le dispositif est prévu et conçu pour mesurer le débit volumique d'un liquide corporel.

10. Dispositif selon l'une quelconque des revendications précédentes, **caractérisé en ce que** le dispositif est prévu et conçu pour être implanté sous la peau d'un patient.

11. Dispositif selon l'une quelconque des revendications précédentes, **caractérisé en ce que** la première soupape (10) et la deuxième soupape (11) sont conçues de telle sorte que le passage de leur position fermée à leur position ouverte et inversement se produise brusquement.

12. Ensemble de mesure du débit volumique d'un fluide s'écoulant dans un conduit, **caractérisé par**
• un dispositif (1) selon la revendication 2 ou un dispositif selon l'une quelconque des revendications 3 à 11 dans la mesure où elles sont subordonnées à la revendication 2,
• un microphone disposé à une distance du dispositif (1) telle qu'il puisse détecter un signal acoustique produit par le dispositif (1) et
• une unité d'évaluation qui coopère avec le microphone et qui est prévue et conçue pour convertir en un débit volumique un signal électrique produit par le microphone.

13. Ensemble selon la revendication 12, **caractérisé en ce que** l'unité d'évaluation comprend une unité de détection de bruit, une unité de comptage et une unité de calcul.

14. Procédé de mesure du débit volumique d'un fluide s'écoulant dans un conduit par recours à un dispositif selon l'une quelconque des revendications 1 à 11, le procédé étant **caractérisé par** les étapes suivantes :
• détection de plusieurs signaux produits par le dispositif (1) lors de chaque fermeture et/ou ouverture d'une première soupape (10) et/ou d'une deuxième soupape (11),
• détermination du nombre des signaux détectés par unité de temps et
• calcul du débit volumique sur la base du nombre des signaux détectés par unité de temps et sur la base de données qui indiquent le volume d'un fluide qui s'écoule dans le dispositif (1) au cours de l'intervalle de temps qui s'écoule entre l'ouverture de la première soupape (10) et/ou de la deuxième soupape (11) et la fermeture de la première soupape (10) et/ou de la deuxième soupape (11).
